(19) Europäisches Patentamt

European Patent Office

Office européen des brevets

(11) Numéro de publication: **0 276 176 B1**

(12) **FASCICULE DE BREVET EUROPEEN**

(45) Date de publication de fascicule du brevet: **02.12.92**    (51) Int. Cl.⁵: **B01D 53/22**, F23L 15/04, F24H 1/44

(21) Numéro de dépôt: **88400015.9**

(22) Date de dépôt: **05.01.88**

(54) **Procédé d'échange simultané de chaleur et de matière à travers une paroi poreuse.**

(30) Priorité: **13.01.87 FR 8700359**

(43) Date de publication de la demande:
**27.07.88 Bulletin 88/30**

(45) Mention de la délivrance du brevet:
**02.12.92 Bulletin 92/49**

(84) Etats contractants désignés:
**BE DE GB IT NL**

(56) Documents cités:
DE-A- 3 344 917     US-A- 2 478 617
US-A- 3 739 553     US-A- 4 040 804
US-A- 4 101 294     US-A- 4 444 571

(73) Titulaire: **INSTITUT FRANCAIS DU PETROLE**
**4, Avenue de Bois Préau**
**F-92502 Rueil-Malmaison(FR)**

(72) Inventeur: **Rojey, Alexandre**
**29-33 rue Henri Regnault**
**F-92380 Garches(FR)**
Inventeur: **Grehier, Alain**
**26, rue Mousset Robert**
**F-75012 Paris(FR)**

## Description

Le procédé selon l'invention s'applique à un échange simultané de chaleur et de matière.

Il peut être utilisé notamment pour effectuer un échange entre un gaz chaud et humide qui est refroidi en transférant de la chaleur et de l'eau à un gaz plus froid et plus sec, qui se charge en vapeur d'eau en se réchauffant.

Un tel échange simultané de chaleur et de matière a déjà été effectué de différentes manières dans l'art antérieur.

Ainsi, le brevet US-A-4,040,804 décrit un procédé et un dispositif permettant à la fois un échange de chaleur et d'humidité à travers une paroi perméable à l'eau ou de papier déliquescent. Un courant d'air relativement chaud et humide (I) et un courant d'air relativement froid et sec (II) circulent à contre-courant de part et d'autre de cette membrane. La nature déliquescente de la barrière perméable permet le transfert d'eau du gaz humide (I) dans le gaz sec (II) au-dessous des conditions de saturation. De ce fait, la totalité de l'eau sous forme vapeur abandonnée par le gaz chaud et humide (I) se trouve restituée quantitativement, sous forme vapeur, dans le gaz froid et sec (II).

Dans ces conditions, dans ce procédé, le gain enthalpique du gaz (II) n'est imputable qu'à la perte de chaleur sensible du gaz (I). Or, ce gain pourrait être largement accru si, de plus, une partie de la chaleur latente de l'eau contenue dans le gaz (I) pouvait être transférée au gaz (II).

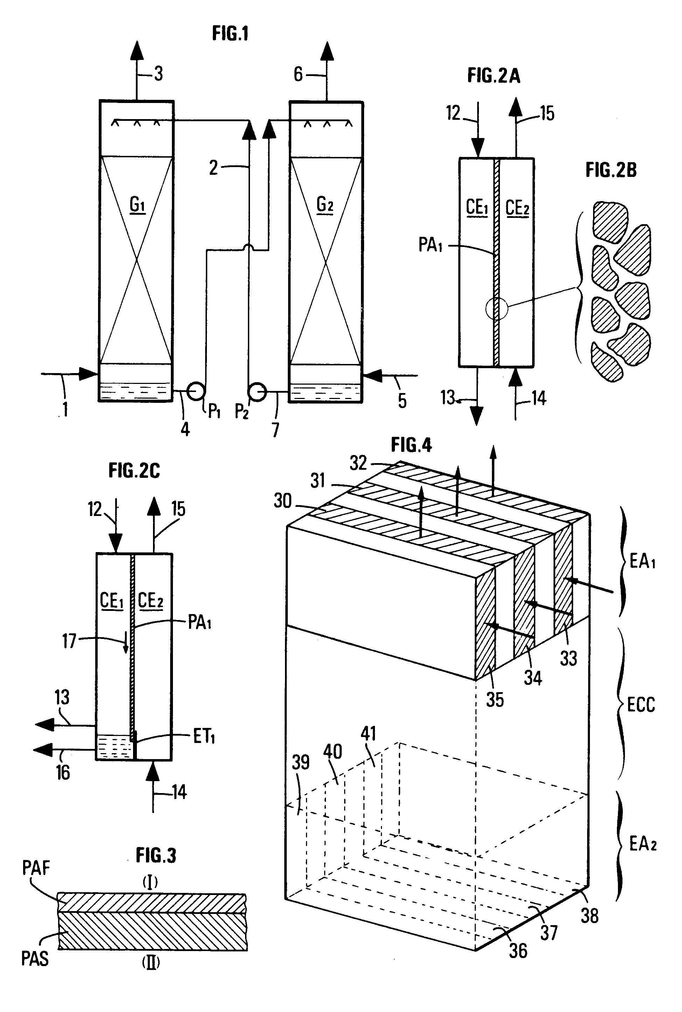

On connait également la demande de brevet FR-A-2.508.616 qui décrit un procédé d'échange simultané de chaleur et de matière dont le principe est décrit ci-après, en liaison avec la figure 1.

Un gaz chaud et humide arrive par le conduit 1 dans une colonne à garnissage G1 dans laquelle il est mis en contact avec un débit d'eau plus froide arrivant par le conduit 2. Le gaz sort refroidi par le conduit 3. Le refroidissement provoque une condensation d'eau supplémentaire qui est évacuée par le conduit 4 et est reprise par la pompe P1 en même temps que l'eau de refroidissement au fond de la colonne à garnissage G1. L'eau ainsi réchauffée est mise en contact dans une colonne à garnissage G2 avec un gaz plus froid et plus sec qui arrive par le conduit 5. Le gaz sort réchauffé par le conduit 6 en se chargeant en eau au cours du contact opéré dans la colonne à garnissage G2.

L'eau recueillie au fond de la colonne à garnissage G2 est évacuée par le conduit 7 et est reprise par la pompe P2.

Ce dispositif présente l'inconvénient d'être encombrant et de nécessiter deux pompes de circulation.

Il a été découvert qu'il est possible d'éviter ces inconvénients en utilisant le procédé selon l'invention.

Ce procédé de transfert simultané de chaleur et de matière d'une phase gazeuse relativement chaude (I) à une phase gazeuse relativement froide (II), la phase gazeuse (I) comprenant au moins, deux constituants A et B ayant des températures de condensation différentes, la température de condensation de A étant plus basse que la température de condensation de B et au moins le constituant B étant au moins en partie condensable dans les conditions du procédé, caractérisé en ce qu'on fait circuler la phase gazeuse (I) au contact de la première face d'une paroi d'échange poreuse, perméable au moins au constituant B à l'état liquide, et présentant une dimension moyenne de pores supérieure à 0,01 mm et une porosité supérieure à 50%, en ce qu'on fait circuler la phase gazeuse (II) au contact de la seconde face de ladite paroi d'échange poreuse, dans une direction sensiblement parallèle et opposée à celle de circulation de la phase gazeuse (I), la température de la phase gazeuse (II) au début du contact étant suffisamment basse pour permettre la condensation d'au moins une fraction du constituant B de la phase gazeuse (I) et le maintien du condensat résultant dans au moins une partie de l'épaisseur de la partie poreuse de la paroi d'échange sur toute la surface de celle-ci, la température de la phase gazeuse (II) n'étant cependant pas trop basse pour éviter une condensation totale des constituants A et B de ladite phase gazeuse (I) au cours dudit contact, les conditions de pression de part et d'autre de la paroi étant mutuellement adaptées pour permettre ladite condensation d'une fraction du constituant B sur ladite première face et la vaporisation de ladite fraction condensée sur ladite seconde face, en ce qu'on laisse retomber par gravité et évacue la phase condensée n'ayant pas traversé ladite paroi et en ce qu'on soutire séparément la phase gazeuse (I) de température abaissée, de concentration en constituant B abaissée et de concentration en constituant A accrue et la phase gazeuse (II) de température et de concentration en constituant B accrues.

Le principe du procédé est décrit en relation avec le schéma de la Figure 2A.

Une phase gazeuse (I) comportant au moins deux constituants A et B arrive par le conduit 12 dans un compartiment d'échange CE1 séparé d'un deuxième compartiment d'échange CE2 par une paroi d'échange PA1 comportant un réseau de pores communiquant entre eux et débouchant de part et d'autre de la paroi selon une configuration telle que celle qui est schématisée sur la Figure 2B.

Le mélange gazeux est refroidi au contact de la paroi PA1. Le constituant B est supposé au moins partiellement condensable dans les conditions de température et de pression ainsi rencontrées par la phase gazeuse (I) et il se forme ainsi une phase liquide au contact de la paroi d'échange PA1 qui remplit sur au moins une partie de l'épaisseur le réseau poreux de la paroi d'échange PA1.

Deux cas peuvent se produire :

- Si le ou les constituant(s) autre(s) que B présent(s) dans le mélange (constituant A) n'est pas (ne sont pas) condensable(s), la phase liquide est formée par le constituant B pratiquement pur. C'est le cas par exemple si le mélange considéré est un mélange d'air et de vapeur d'eau évoluant dans des conditions proches de l'ambiance.
- Si un constituant autre que B présent dans le mélange (constituant A) est au moins partiellement condensable dans les conditions de température et de pression rencontrées au cours du refroidissement au contact de la paroi PA1, tout en étant plus volatil que le constituant B, la phase liquide obtenue est formée par un mélange comportant les constituants A et B mais plus riche en constituant B que le mélange gazeux de départ.

Dans tous les cas, la phase gazeuse (I) sort du compartiment CE1 par le conduit 13 refroidie et avec une concentration en constituant B plus basse qu'à l'entrée.

Le refroidissement de la phase gazeuse (I) au contact de la paroi PA1 est assuré en faisant circuler dans le compartiment CE2 une phase gazeuse (II) qui entre dans le compartiment CE2 par le conduit 14 à une température plus basse que la température d'entrée dans le compartiment CE1 de la phase gazeuse (I).

La phase gazeuse (II) ressort du compartiment CE2 par le conduit 15 réchauffée par échange de chaleur avec la phase gazeuse (I) à travers la paroi PA1 en entraînant à l'état de vapeur au moins une partie du constituant B ayant condensé dans le réseau poreux de la paroi d'échange.

Sur le schéma de la Figure 2A n'a été représentée qu'une paroi d'échange, mais il est clair que dans le procédé selon l'invention, le dispositif utilisé pour effectuer l'échange pourra compter une pluralité de parois et de compartiments en alternance.

De même différentes géométries peuvent être mises en oeuvre, les parois pouvant être par exemple planes ou cylindriques ou comporter des ondulations ou des nervures.

La ou les paroi(s) poreuse(s) séparant les phases gazeuses (I) et (II) sont caractérisées par une porosité relativement élevée, par exemple supérieure à 50 % et une dimension moyenne des pores généralement très grande par rapport à la dimension des molécules formant les phases gazeuses (I) et (II), par exemple supérieure à 0,01 mm. Par conséquent, en l'absence de phase liquide on n'observe aucune sélectivité vis-à-vis du constituant B, c'est-à-dire qu'un mélange de constituants A et B traversant la paroi poreuse non remplie de liquide sous l'effet d'une différence de pression ressort avec pratiquement la même composition.

La présence du film liquide de condensat est donc essentielle pour effectuer un échange de matière sélectif.

Il est par conséquant important que la ou les paroi(s) poreuse(s) séparant les phases gazeuses (I) et (II) soient remplies de liquide sur au moins une partie de l'épaisseur sur toute la surface en contact avec chacune des phases gazeuses (I) et (II).

Un exemple de disposition permettant d'assurer une telle condition est schématisé sur la Figure 2C qui reprend la disposition de base de la Figure 2A.

La paroi PA1 est verticale et se remplit de phase liquide par capillarité. Elle communique à sa partie inférieure avec une réserve de phase liquide qui est maintenue à un niveau constant en évacuant par le conduit 16 l'excédent de phase liquide condensé qui s'écoule le long de la paroi (écoulement schématisé par la flèche 17). L'étanchéité est assurée à la partie inférieure de la paroi PA1, en contact avec la réserve de phase liquide, par la plaque d'étanchéité ET1, la hauteur de la plaque ET1 étant supérieure à la hauteur de paroi PA1 en contact avec la réserve liquide.

Les phases gazeuses (I) et (II) sont admises dans les compartiments CE1 et CE2 à des pressions voisines.

Une différence de pression de part et d'autre de la paroi est toutefois acceptable tant qu'elle reste inférieure à la surpression capillaire qui maintient la phase liquide condensée dans les pores, cette surpression étant d'autant plus élevée que le diamètre moyen des pores est réduit, que la tension superficielle de la phase liquide condensée est grande et que l'angle de contact entre ladite phase liquide et le matériau constituant la paroi poreuse est petit, ledit angle devant toujours être inférieur à 90°.

Différents matériaux peuvent être utilisés pour réaliser la paroi poreuse PA1.

La paroi poreuse PA1 peut être réalisée par exemple par agglomération de particules.

Ces particules peuvent être constituées de matériaux différents : métaux, céramiques, polymères et avoir différentes géométries, par exemple particules sensiblement sphériques en poudre ou fibres allongées.

La paroi poreuse PA1 peut être ainsi réalisée par frittage de poudres métalliques, le métal utilisé pouvant être par exemple du cuivre, de l'aluminium, du nickel ou de l'acier.

Elle peut être également réalisée en mettant en oeuvre des feutres de fibres non tissées, ces fibres pouvant être par exemple des fibres de verre ou des fibres de matériau polymère, notamment des fibres de polymère textile tel que le polyester.

Pour obtenir des coefficients de transfert thermique élevés, il est avantageux de réduire l'épaisseur du film liquide qui se forme dans la paroi poreuse.

Ceci peut être obtenu en limitant l'épaisseur de la paroi poreuse PA1 à une valeur inférieure par exemple à 5 mm et de préférence inférieure à 2 mm.

Dans ce cas et notamment si le matériau poreux utilisé est un feutre il est avantageux pour améliorer la résistance mécanique de la paroi, de la supporter par une grille qui peut être métallique ou en matériau polymère, ou un support très poreux qui ne se remplit pas de liquide.

Ce dernier cas est schématisé sur la Figure 3.

Dans ce cas, la paroi poreuse PA1 est obtenue en juxtaposant une paroi poreuse PAF mouillée par la phase liquide condensée, d'épaisseur réduite, de préférence inférieure à 2 mm, en contact avec la phase gazeuse (I) et une paroi poreuse PAS non mouillée par la phase liquide condensée.

La paroi poreuse PAS est caractérisée par une porosité élevée et une dimension relative des pores importante, par exemple de l'ordre de 1 mm. Elle est réalisée de préférence en un matériau non mouillable par la phase liquide condensée.

La paroi poreuse peut n'être poreuse que sur une partie de sa surface.

Pour améliorer l'efficacité de l'échange simultané de chaleur et de matière effectué entre les phases gazeuses (I) et (II), il est avantageux d'utiliser un dispositif multi-canaux tel que celui qui est schématisé sur la Figure 4.

Le dispositif est constitué de plaques planes de séparation qui répondent aux conditions décrites précédemment.

Dans les compartiments situés entre ces plaques planes circulent alternativement la phase gazeuse (I) et la phase gazeuse (II).

Le dispositif comporte trois zones d'échange :
- Dans la zone d'échange ECC les deux phases circulent à contre-courant.
- Dans la zone d'admission EA1 la phase gazeuse (I) sort par les orifices 30, 31 et 32, les orifices adjacents étant bouchés pour éviter tout passage de la phase gazeuse (II). La phase gazeuse (II) entre par les orifices 33, 34 et 35, les orifices adjacents étant bouchés pour éviter tout passage de la phase gazeuse (I).
- Dans la zone d'admission EA2, la phase gazeuse (I) entre par les orifices 36, 37 et 38, les orifices adjacents étant bouchés pour éviter tout passage de la phase gazeuse (II). La phase gazeuse (II) sort par les orifices 39, 40 et 41, les orifices adjacents étant bouchés pour éviter tout passage de la phase gazeuse (I).

Le procédé selon l'invention peut être utilisé pour effectuer un transfert simultané de chaleur et d'eau entre deux gaz.

Différentes applications peuvent être envisagées.

Il est possible notamment de réaliser un tel échange entre l'air froid et sec provenant de la ventilation d'une enceinte à air conditionné et l'air chaud et humide qui est envoyé vers l'enceinte avant qu'il ne soit réfrigéré afin de réduire la consommation d'énergie du groupe de réfrigération.

Un tel échange simultané de chaleur et d'eau peut être envisagé d'autre part dans le cas de phases gazeuses de natures très différentes.

Ainsi il est possible d'effectuer un échange de chaleur et d'eau entre un gaz naturel chaud et humide avant une étape de réfrigération destinée à recueillir une fraction liquide et le gaz naturel froid et sec provenant de l'étape de réfrigération après séparation de la fraction liquide.

Le constituant condensable peut être autre que l'eau. Ainsi il est possible de récupérer un solvant présent dans l'air chaud d'une enceinte dans laquelle ce solvant est mis en oeuvre en effectuant un échange avec de l'air froid dépourvu de solvant envoyé à l'enceinte.

Une partie de la phase gazeuse (I) sortant du dispositif d'échange refroidie et appauvrie en constituant B peut être refroidie à l'extérieur du dispositif d'échange et renvoyée au dispositif d'échange pour former la phase gazeuse (II).

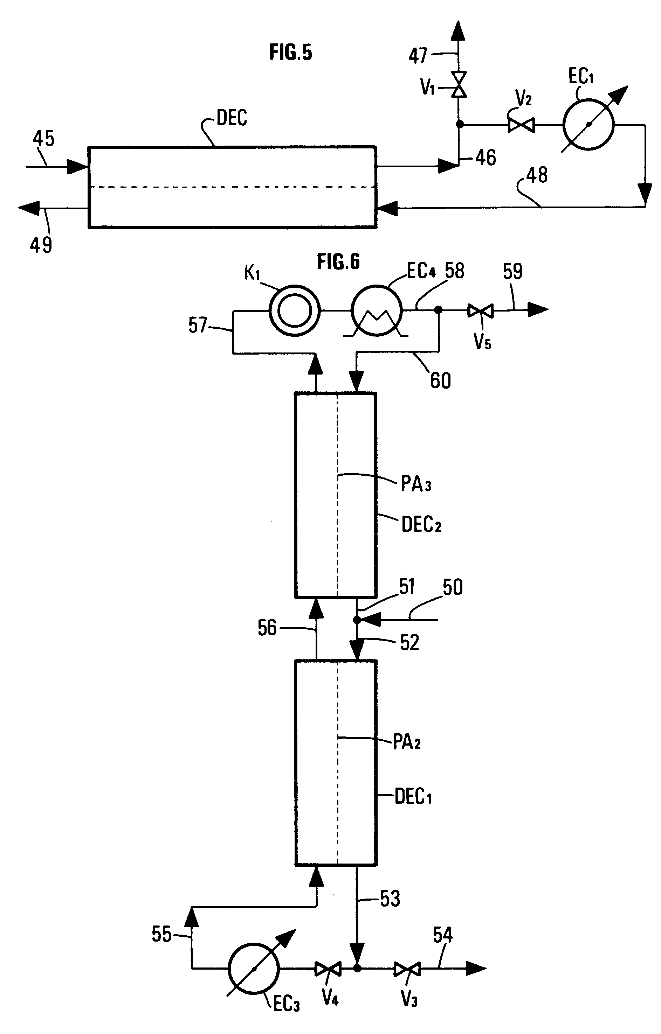

On opère dans ce cas selon le schéma représenté sur la Figure 5.

Le gaz chaud formé par le mélange des constituants A et B arrive par le conduit 45 dans le dispositif d'échange DEC, dans lequel il forme la phase gazeuse (I). Il en ressort refroidi, appauvri en constituant B et enrichi en constituant A par le conduit 46. Une partie du gaz est évacuée par le conduit 47, le débit de gaz évacué étant réglé par la vanne V1. La fraction de gaz non évacuée est détendue à travers la vanne V2, refroidie dans l'échangeur EC1 et renvoyée par le conduit 48 vers le dispositif d'échange DEC dans lequel elle forme la phase gazeuse (II). Elle en ressort par le conduit 49 en entraînant au moins en partie le constituant B présent dans le mélange initial.

Il est possible d'augmenter la concentration en constituant B dans le gaz sortant par le conduit 49 en abaissant la pression, ce qui permet de réduire la fraction de gaz recyclée. Il faut dans ce cas respecter l'écart de pression maximum que peut tolérer le film de liquide qui se forme dans les parois d'échange poreuses du dispositif d'échange DEC, compte tenu de la surpression capillaire.

Un tel mode d'échange permet de fractionner un mélange de constituants condensables lors de l'échange.

Le schéma représenté sur la Figure 6 illustre l'agencement qui peut être mis en oeuvre dans ce cas. On supposera pour décrire la méthode de séparation que le mélange ne comprend que deux constituants A et B condensables, B étant le constituant dont la température d'ébullition est la plus élevée, mais la méthode peut être également utilisée pour séparer un mélange de plus de deux constituants en deux fractions distinctes.

Le mélange de constituants A et B arrive par le conduit 50. Il est mélangé avec le débit vapeur qui arrive par le conduit 51 et le mélange résultant est envoyé par le conduit 52 au dispositif d'échange DEC1. Le dispositif d'échange DEC1 comporte des parois poreuses telles que décrites précédemment, dans lesquelles il se forme un film liquide dont la composition évolue entre l'entrée et la sortie du mélange vapeur qui arrive par le conduit 52. Cette composition s'enrichit en constituant A au fur et à mesure que la température diminue et la phase vapeur recueillie par le conduit 53 à la sortie du dispositif d'échange DEC1 peut être formée par le constituant A presque pur. Une fraction de cette phase vapeur est évacuée par le conduit 54, la vanne V3 permettant de régler le débit évacué. La fraction restante est détendue à travers la vanne V4, refroidie dans l'échangeur EC3 puis envoyée par le conduit 55 au dispositif d'échange DEC1. Elle ressort du dispositif d'échange DEC1, réchauffée, enrichie en constituant B, par le conduit 56, et est envoyée au dispositif d'échange DEC2. Le dispositif d'échange DEC2 comporte des parois poreuses telles que décrites précédemment dans lesquelles il se forme un film liquide dont la composition évolue entre l'entrée et la sortie du mélange vapeur qui arrive par le conduit 56. La composition du gaz s'enrichit en constituant B entre l'entrée 56 et la sortie 57 en même que la température augmente. La phase vapeur recueillie par le conduit 57 à la sortie du dispositif d'échange DEC2 peut être ainsi formée par le constituant B presque pur. Cette phase vapeur est comprimée dans le compresseur (surpresseur) K1 puis chauffée dans l'échangeur EC4. L'étape de chauffage dans l'échangeur EC4 peut ne pas être nécessaire, l'élévation de température obtenue au cours de l'étape de compression dans le compresseur K1 pouvant être suffisante. Une fraction de la phase vapeur comprimée et réchauffée est évacuée, la vanne V5 permettant de régler le débit évacué. La fraction restante est envoyée par le conduit 60 au dispositif d'échange DEC2. Elle ressort par le conduit 51 du dispositif d'échange DEC2 refroidie, appauvrie en constituant B et enrichie en constituant A.

Il est possible ainsi de séparer le mélange de constituants A et B qui arrive par le conduit 50 en une fraction formée essentiellement par le constituant A, qui est évacuée par le conduit 54, et une fraction formée essentiellement par le constituant B, qui est évacuée par le conduit 59.

EXEMPLE 1

L'exemple 1 est décrit en relation avec la Figure 2C.

On introduit dans le compartiment CE1, par le conduit 12, un débit de 111,43 kg/h d'air humide (fluide A) constitué de 100 kg/h d'air sec et de 11,43 kg/h d'eau, à la température de 55 °C. On envoie simultanément dans le compartiment CE2, par le conduit 14, un débit de 100,74 kg/h d'air humide (fluide B) constitué de 100 kg/h d'air sec et de 0,74 kg/h d'eau, à la température de 15 °C de telle sorte que les fluides A et B circulent à contre-courant de part et d'autre d'une membrane poreuse PA1 dont toute la porosité est imprégnée d'eau sous forme liquide.

La membrane PA1 est constituée dans cet exemple d'un feutre non tissé ayant une épaisseur voisine de 1 millimètre constitué de fibres de cellulose.

Lors du passage dans le compartiment CE1, le fluide A se refroidit au contact de la membrane PA1 en abandonnant par condensation sur celle-ci une partie de l'eau qu'il contient tandis que le fluide B, circulant dans le compartiment CE2, se réchauffe et se charge d'eau au contact de la membrane PA1. Les échanges de chaleur et d'eau entre les fluides A et B sont tels que, d'une part, le fluide A sort par le conduit 13 à la température de 42 °C et est constitué de 100 kg/h d'air sec et de 5,53 kg/h d'eau sous forme vapeur tandis que 0,51 kg/h sont évacués sous forme liquide par le conduit 16 à la température de 28,5 °C et, d'autre part, le fluide B sort par le conduit 15 à la température de 43,6 °C et comprend 100 kg/h d'air sec et 6,13 kg/h d'eau sous forme vapeur.

Dans cet exemple, l'eau condensée en excès est évacuée du compartiment CE1 par gravité. A cet effet, une paroi ET1 non perméable évite le passage de l'eau condensée du compartiment CE1 où circule le fluide A vers le compartiment CE2 où circule le fluide B et permet ainsi son évacuation par gravité par le conduit 16 qui comprend les dispositions nécessaires au maintien d'un tampon liquide à niveau constant à la partie inférieure du compartiment CE1 afin d'éviter l'évacuation du fluide A par le conduit 16.

La puissance calorifique transférée du fluide A vers le fluide B s'élève à 4,4 kW dont environ 4 kW et 0,4 kW et 0,4 kW résultent respectivement de la chaleur de condensation de l'eau issue du fluide A sur la paroi poreuse et de la chaleur sensible due au refroidissement du fluide A. Simultanément, le transfert d'eau du fluide A vers le fluide B s'élève à 5,39 kg/h.

EXEMPLE 2

L'exemple 2 est décrit en relation avec le schéma de la Figure 6. Dans cet exemple, les parois poreuses PA2 et PA3 des dispositifs d'échange DEC1 et DEC2 sont constituées de feuilles de nickel fritté d'épaisseur égale à 0,8 millimètre.

On envoie par le conduit 50 un débit de 1 kg/h d'un mélange gazeux de pentane normal ($nC_5$) et d'heptane normal ($nC_7$) dont la composition en fractions molaires est la suivante :

$nC_5$ : 0,7      $nC_7$ : 0,3

Après mélange avec 6 kg/h d'un gaz provenant du dispositif d'échange DEC2 par le conduit 51, on obtient un mélange en phase vapeur à une température de 70 °C et une pression de 760 mm Hg, qui est envoyé par le conduit 52 au dispositif d'échange DEC1. A la sortie du dispositif d'échange DEC1, on recueille par le conduit 53 du pentane en phase vapeur contenant moins de 1 % d'heptane à une température de 35 °C. Un débit de 0,63 kg/h est prélevé par le conduit 54. La fraction restante est refroidie jusqu'à 28 °C dans l'échangeur EC3 et envoyée en phase vapeur à une pression de 570 mm Hg par le conduit 55 dans le dispositif d'échange DEC1. Elle en ressort par le conduit 56 à une température de 67 °C et est envoyée au dispositif d'échange DEC2. A la sortie du dispositif d'échange DEC2 on recueille par le conduit 57 de l'heptane en phase vapeur contenant moins de 1 % de pentane à une température de 84 °C. Il est comprimé jusqu'à une pression de 800 mm Hg dans le compresseur K1. La phase vapeur est alors envoyée dans l'échangeur EC4 d'où elle ressort par le conduit 58 à une température de 100 °C.

Un débit de 0,37 kg/h est prélevé par le conduit 59. La fraction restante est envoyée en phase vapeur par le conduit 60 au dispositif d'échange DEC2.

Dans cet exemple le mélange de pentane normal et d'heptane normal a été séparé sans qu'il soit nécessaire comme en distillation d'avoir recours à un changement de phase pour obtenir les débits de reflux.

Une condition essentielle dans le mode de réalisation illustré par la Figure 6 est que, lors de chacune des étapes d'échange, le fluide qui se refroidit soit à une pression moyenne plus élevée que le fluide qui se réchauffe.

**Revendications**

1. Procédé de transfert simultané de chaleur et de matière d'une phase gazeuse relativement chaude (I) à une phase gazeuse relativement froide (II), la phase gazeuse (I) comprenant au moins deux constituants A et B ayant des températures de condensation différentes, la température de condensation de A étant plus basse que la température de condensation de B et au moins le constituant B étant au moins en partie condensable dans les conditions du procédé, caractérisé en ce qu'on fait circuler la phase gazeuse (I) au contact de la première face d'une paroi d'échange poreuse perméable au moins au constituant B à l'état liquide, et présentant une dimension moyenne de pores supérieure à 0,01 mm et une porosité supérieure à 50%, en ce qu'on fait circuler la phase gazeuse (II) au contact de la seconde face de ladite paroi d'échange poreuse, dans une direction sensiblement parallèle et opposée à celle de circulation de la phase gazeuse (I), la température de la phase gazeuse (II) au début du contact étant suffisamment basse pour permettre la condensation d'au moins une fraction du constituant B de la phase gazeuse (I) et le maintien du condensat résultant dans au moins une partie de l'épaisseur de la partie poreuse de la paroi d'échange sur toute la surface de celle-ci, la température de la phase gazeuse (II) n'étant cependant pas trop basse pour éviter une condensation totale des constituants A et B de ladite phase gazeuse (I) au cours dudit contact, les conditions de pression de part et d'autre de la paroi étant mutuellement adaptées pour permettre ladite condensation d'une fraction du constituant B sur ladite première face et la vaporisation de ladite fraction condensée sur ladite seconde face, en ce qu'on laisse retomber par gravité et évacue la phase condensée n'ayant pas traversé ladite paroi et en ce qu'on soutire séparément la phase gazeuse (I) de température abaissée, de concentration en constituant B abaissée et de concentration en constituant A accrue et la phase gazeuse (II) de température et de concentration en constituant B accrues.

2. Procédé selon la revendication 1, caractérisé en ce que la phase condensée contenue dans la paroi est renouvelée au moins en partie par capillarité.

3. Procédé selon l'une des revendications 1 et 2, caractérisé en ce que la paroi d'échange poreuse est verticale.

**4.** Procédé selon l'une des revendications 1 à 3, caractérisé en ce que la paroi d'échange poreuse consiste en un aggloméra de particules.

**5.** Procédé selon l'une des revendications 1 à 4, caractérisé en ce que la paroi d'échange poreuse est constituée de fibres en matériau polymère.

**6.** Procédé selon l'une des revendications 1 à 5, caractérisé en ce que la paroi d'échange poreuse a une épaisseur inférieure à 5 mm.

**7.** Procédé selon l'une des revendications 1 à 6, caractérisé en ce que le constituant B est de l'eau et le constituant A est un gaz non condensable dans les conditions de température et de pression rencontrées au cours de l'échange.

**8.** Procédé selon l'une des revendications 1 à 6, caractérisé en ce que les constituants A et B sont tous deux condensables dans les conditions du procédé.

**9.** Procédé selon l'une des revendications 1 à 8, caractérisé en ce que l'on divise la phase gazeuse (I) soutirée en au moins deux fractions, en ce qu'on refroidit davantage une première fraction (Ia) de la phase gazeuse (I) divisée, en ce quon la fait ensuite circuler au contact de la seconde face de la paroi d'échange, ladite première fraction constituant ainsi au moins une partie de la phase gazeuse (II), et en ce qu'on décharge une seconde fraction (Ib) de la phase gazeuse (I) divisée.

**10.** Procédé selon la revendication 8, caractérisé en ce que l'on divise la phase gazeuse (I) soutirée en au moins deux fractions, en ce qu'on évacue une première fraction (Ia), et en ce qu'on refroidit davantage la fraction restante (Ib) de la phase gazeuse (I) divisée, en ce qu'on abaisse sa pression, en ce qu'on la fait ensuite circuler au contact de la seconde face de la paroi d'échange, ladite fraction (Ib) constituant ainsi au moins une partie de la phase gazeuse (II), en ce qu'on fait circuler la phase gazeuse (II), après son contact avec ladite seconde face de la paroi d'échange, au contact de la première face d'une seconde paroi d'échange perméable au moins au constituant B à l'état liquide, en ce quon chauffe et élève la pression de la phase gazeuse (III) obtenue après ledit contact, en ce qu'on divise la phase gazeuse chauffée et comprimée en une première fraction (IIIa), que l'on évacue, en ce qu'on fait ensuite circuler la fraction restante (IIIb) au contact de la seconde face de la seconde paroi d'échange dans une direction sensiblement parallèle et opposée à celle de la phase gazeuse (II), la température de la phase gazeuse (II) au début du contact avec la première face de la seconde paroi d'échange étant suffisamment basse pour permettre la condensation d'au moins une fraction du constituant B de la seconde fraction gazeuse (IIIb) et le maintien du condensat résultant dans au moins une partie de l'épaisseur de la seconde paroi d'échange, ladite température n'étant cependant pas trop basse pour éviter une condensation totale des constituants A et B de ladite seconde fraction de phase gazeuse (IIIb) au cours dudit contact, les conditions de pression de part et d'autre de la seconde paroi d'échange étant mutuellement adaptées pour permettre ladite condensation d'une fraction du constituant B sur ladite seconde face et la vaporisation de ladite fraction condensée sur ladite première face de ladite seconde paroi, et en ce qu'on mélange la phase gazeuse (IV) résultant dudit contact avec la phase gazeuse relativement chaude (I) soumise au procédé.

**11.** Procédé selon la revendication 7, caractérisé en ce que le constituant A consiste essentiellement en de l'air ou du gaz naturel.

**12.** Procédé selon la revendication 10, caractérisé en ce qu'il est appliqué à la séparation de deux hydrocarbures gazeux.

**Claims**

**1.** A process for the simultaneous transfer of heat and matter from a relatively hot gaseous phase (I) to a relatively cold gaseous phase (II), the gaseous phase (I) comprising at least two constituents A and B having different condensation temperatures, the condensation temperature of A being lower than the condensation temperature of B and at least the constituent B being at least in part condensable under the conditions of the process, characterized in that the gaseous phase (I) is circulated in contact with the first face of a porous exchange wall permeable to at least the constituent B in the liquid state, and having an average size of pores higher than 0.01 mm and a porosity higher than 50%, in that the gaseous phase (II) is circulated in contact with the second face of said porous exchange wall, in a direction substantially parallel and opposite to that of the circulation of the gaseous phase (I), the temperature of the gaseous phase (II) at the beginning of the contact being sufficiently low to permit the condensation of at least a fraction of the constituent B of the gaseous phase (I) and the maintenance of the resulting condensate in at least a part of the thickness of the porous portion of the exchange wall over the whole surface thereof, the temperature of the gaseous phase (II) not however being too low to avoid total condensation of the constituents A and B of said gaseous phase (I) in the course of said contact, the conditions of pressure on each side of the wall being mutually adapted to permit said condensation of a fraction of the constituent B on the said first face and the vaporisation of said condensed fraction on said second face, in that the condensed phase which has not transferred through said wall is allowed to fall down by gravity, and in that the gaseous phase (I) of lowered temperature, of lowered concentration of constituent B and of increased concentration of constituent A and the the gaseous phase (II) of increased temperature and increased concentration of constituent B are withdrawn separetely.

2.  A process according to claim 1, characterized in that the condensed phase contained in the wall is renewed at least in part by capillarity.

3.  A process according to one of claims 1 and 2, characterized in that the porous exchange wall is vertical.

4.  A process according to one of claims 1 to 3, characterized in that the porous exchange wall consists of an agglomerate of particles.

5.  A process according to one of claims 1 to 4, characterized in that the porous exchange wall is constituted by fibers of a polymer material.

6.  A process according to one of claims 1 to 5, characterized in that the porous exchange wall has a thickness lower than 5 mm.

7.  A process according to one of claims 1 to 6, characterized in that the constituent B is water and the constituent A is a gas not condensable under the conditions of temperature and pressure encountered in the course of the exchange.

8.  A process according to one of claims 1 to 6, characterized in that the constituent A and B are both condensable under the conditions of the process.

9.  A process according to one of claims 1 to 8, characterized in that the gaseous phase (I) withdrawn is divided into at least two fractions, in that a first fraction (Ia) of the divided gaseous phase (I) is cooled in the greater extent, then circulated in contact with the second face of the exchange wall, said first fraction thus constituing at least a portion of the gaseous phase (II), and in that a second fraction (Ib) of the divided gaseous phase (I) is discharged.

10. A process according to claim 8, characterized in that the gaseous phase (I), withdrawn is dividedin at least two fractions, in that a first fraction (Ia) is withdrawn and in that the remaining fraction (Ib) of the divided gaseous phase (I) is cooled in a greater extent, in that its pressure is lowered, in that it is then circulated in contact with the second face of the exchange wall, said fraction (Ib) thus constituting at least a portion of the gaseous phase (II), in that the the gaseous phase (II) is circulated after its contact with said second face of the exchange wall, in contact with the first face of a second exchange wall permeable at least to the constituent B in the liquid state, in that the gaseous phase (III) obtained after said contact is heated and its pressure raised, in that the gaseous phase heated and comprimed is divided into a first fraction (IIIa), which is discharged, in that the remaining fraction (IIIb) is then circulated in contact with the second face of the second exchange wall in a direction substantially parallel and opposite to that of the gaseous phase (II), the temperature of the gaseous phase (II) at the start of the contact with the first face of the second exchange wall being sufficiently low to permit the condensation of at least a fraction of the constituent B of the second gaseous fraction (IIIb) and maintenance of the resulting condensate in at least a part of the thickness of the second exchange wall, said temperature not however being too low to avoid a total condensation of the constituents A and B of said second fraction of gaseous phase (IIIb) in the course of said contact, the conditions of pressure on each side of the second exchange wall being mutually adapted to permit said condensation of a fraction of the constituents B on said second face and vaporisation of said condensed fraction on said first face of said second wall, and in that the gaseous phase (IV) resulting from said contact is mixed with the relatively hot gaseous phase (I) subjected to the process.

11. A process according to claim 7, characterized in that the constituent A is essentially air or natural gas.

**12.** A process according to claim 10, characterized in that it is applied to the separation of two gaseous hydrocarbons.

**Patentansprüche**

**1.** Verfahren zur gleichzeitigen Übertragung von Wärme und Material einer gasförmigen relativ heißen Phase (I) an eine gasförmige relativ kalte Phase (II), wobei die Gasförmige Phase (I) wenigstens zwei Bestandteile A und B, die unterschiedliche Kondensationstemperaturen aufweisen, umfaßt, wobei die Kondensationstemperatur von A niedriger als die Kondensationstemperatur von B ist und wenigstens der Bestandteil B zum Teil unter den Verfahrensbedingungen kondensierbar ist, dadurch gekennzeichnet, daß man die gasförmige Phase (I) in Kontakt mit der ersten Fläche einer porösen Austauscherwand, die permeabel wenigstens für den Bestandteil B im flüssigen Zustand ist, zirkulieren läßt, wobei diese eine mittlere Porenabmessung von mehr als 0,01 mm und eine Porösität von mehr als 50 % aufweist, daß man die gasförmige Phase (II) in Kontakt mit der zweiten Fläche dieser porösen Austauscherwand in einer Richtung im wesentlichen parallel und entgegengesetzt zur Zirkulation der gasförmigen Phase (I) zirkulieren läßt, wobei die Temperatur der gasförmigen Phase (II) zu Beginn des Kontakts ausreichend niedrig ist, um die Kondensation wenigstens einer Franktion des Bestandteils B der gasförmigen Phase (I) sowie das Halten des resultierenden Kondensats in wenigstens eine Teil der Dicke des porösen Teils der Austauscherwand über deren gesamte Fläche zuzulassen, wobei die Temperatur der gasförmigen Phase (II) nicht zu niedrig ist, um eine totale Kondensation der Bestandteile A und B dieser gasförmigen Phase(I) während dieses Kontaktes zu vermeiden, wobei die Druckbedingungen zu zu beiden Seiten der Wand gegenseitig angepaßt sind, um diese Kondensation einer Fraktion des Bestandteils B auf dieser ersten Fläche und die Verdampfung dieser kondensierten Fraktion auf dieser zweiten Fläche zu ermöglichen, daß man durch Schwerkraft die kondensierte Phase, welche diese Wand nicht durchsetzt hat, zurückfallen läßt und sie evakuiert und daß man getrennt die gasförmige Phase (I) von abgesenkter Temperatur, von einer abgesenkten Konzentration an Bestandteil B und einer gesteigerten Konzentration an Bestandteil A und die gasförmige Phase (II) von gesteigerter Temperatur und gesteigerter Konzentration am Bestandteil B abzieht.

**2.** Verfahren nach Anspruch 1, dadurch gekennzeichnet, daß die in der Wandung enthaltene kondensierte Phase wenigstens zum Teil durch Kapillarität erneuert wird.

**3.** Verfahren nach einem der Ansprüche 1 und 2, dadurch gekennzeichnet, daß die poröse Austauscherwandung vertikal ist.

**4.** Verfahren nach einem der Ansprüche 1 bis 3, dadurch gekennzeichnet, daß die poröse Austauscherwandung aus einem Partikelagglomerat besteht.

**5.** Verfahren nach einem der Ansprüche 1 bis 4, dadurch gekennzeichnet, daß die poröse Austauscherwandung gebildet wird durch Fasern aus Polymermaterial.

**6.** Verfahren nach einem der Ansprüche 1 bis 5, dadurch gekennzeichnet, daß die poröse Austauscherwandung eine Dicke von weniger als 5 mm hat.

**7.** Verfahren nach einem der Ansprüche 1 bis 6, dadurch gekennzeichent, daß der Bestandteil B Wasser und der Bestandteil A ein Gas ist, das unter den während des Austausches auftretenden Temperatur- und Druckbedingungen nichtkondensierbar ist.

**8.** Verfahren nach einem der Ansprüche 1 bis 6, dadurch gekennzeichnet, daß die Bestandteile A und B beide unter den Verfahrensbedingungen kondensierbar sind.

**9.** Verfahren nach einem der Ansprüche 1 bis 8, dadurch gekennzeichnet, daß man die gasförmige abgezogene Phase(I) in wenigstens zwei Fraktionen unterteilt, daß man um so stärker eine erste Fraktion (Ia) der abgetrennten gasförmigen Phase (I) kühlt, daß man dann in Kontakt mit der zweiten Fläche der Austauscherwandung die erste Fraktion zirkulieren läßt, die so wenigstens einen Teil der gasförmigen Phase (II) bildet, und daß man eine zweite Franktion (Ib) der gasförmigen geteilten Phase (I) austrägt.

**10.** Verfahren nach Anspruch 8, dadurch gekennzeichnet, daß man die gasförmige abgezogene Phase (I) in wenigstens zwei Fraktionen unterteilt, daß man eine erste Fraktion (Ia) abzieht und daß man um so stärker die verbleibende Fraktion (Ib) der gasförmigen geteilten Phase (I) kühlt, daß man ihren Druck absenkt, daß man sie dann in Kontakt mit der zweiten Phase der Austauscherwand zirkulieren läßt, wobei diese Fraktion (Ib) so wenigstens einen Teil der gasförmigen Phase (II) bildet, daß man die gasförmige Phase (II) nach ihrem Kontakt mit dieser zweiten Fläche der Austauscherwand in Kontakt mit der ersten Fläche einer zweiten Austauscherwand zirkulieren läßt, die permeabel wenigstens für den Bestandteil B im flüssigen Zustand ist, daß man erwärmt und den Druck der gasförmigen nach diese Kontakt erhaltenen Phase (III) erhöht, daß man die erwärmte und komprimierte gasförmige Phase unterteilt in eine erste Fraktion (IIIa), die man evakuiert, daß man dann die verbleibende Fraktion (IIIb) in Kontakt mit der zweiten Fläche der zweiten Austauscherwand in einer Richtung im wesentlichen parallel und entgegengesetzt zu der der gasförmigen Phase (II) zirkulieren läßt, wobei die Temperatur der gasförmigen Phase (II) zu Beginn des Kontakts mit der ersten Fläche der zweiten Austauscherwand ausreichend niedrig ist, um die Kondensation wenigstens einer Fraktion des Bestandteils B der zweiten gasförmigen Phase (IIIb) und das Halten des resultierenden Kondensats in wenigstens eine Teil der Dicke der zweiten Austauscherwand zuzulassen, wobei diese Temperatur jedoch nicht zu niedrig ist, um eine totale Kondensation der Bestandteile A und B dieser zweiten Fraktion gasförmiger Phase (IIIb) während dieses Kontaktes zu vermeiden, wobei die Druckbedingungen zu beiden Seiten der zweiten Austauscherwand gegenseitig angepaßt sind, un diese Kondensation einer Fraktion des Bestandteils B auf dieser zweiten Fläche sowie die Verdampfung dieser kondensierten Fraktion auf dieser ersten Fläche dieser zweiten Wand zuzulassen und daß man die aus diesen Kontakt resultierende gasförmige Phase (IV) mit der relativ heißen (I) dem Verfahren ausgesetzten gasförmigen Phase vermischt.

11. Verfahren nach Anspruch 7, dadurch gekennzeichnet, daß der Bestandteil A im wesentlichen aus Luft oder Erdgas besteht.

12. Verfahren nach Anspruch 10, dadurch gekennzeichnet, daß es angewendet wird auf die Trennung von zwei gasförmigen Kohlenwasserstoffen.

## FIG.1

## FIG.2A

## FIG.2B

## FIG.2C

## FIG.4

## FIG.3

**FIG.5**

**FIG.6**